# FASCICULE DE BREVET EUROPEEN

(11) **EP 3 058 341 B1**
(45) Date de publication et mention de la délivrance du brevet: **16.05.2018**
(21) Numéro de dépôt: 14824867.7
(22) Date de dépôt: 17.10.2014
(51) Int. Cl.: G01N 21/27, G01N 21/35, G01N 33/04, G01N 33/49, A61B 5/00, G01N 21/31, C12Q 1/32, G01N 33/50, G01N 21/3577, G01N 33/487

(54) **DETERMINATION DE LA CONCENTRATION D'UN COMPOSANT DANS UN FLUIDE D'UN ANIMAL PAR ANALYSE SPECTROSCOPIQUE D'UN AUTRE FLUIDE**
BESTIMMUNG DER KONZENTRATION EINER KOMPONENTE IN EINEM FLUID EINES TIERES DURCH SPEKTRALANALYSE EINES ANDEREN FLUIDS
DETERMINATION OF THE CONCENTRATION OF A COMPONENT IN ONE FLUID OF AN ANIMAL BY SPECTROSCOPIC ANALYSIS OF ANOTHER FLUID

(30) Priorité: 18.10.2013 FR 1360145
(43) Date de publication de la demande: 24.08.2016
(73) Titulaire: Bentley Instruments S.a.r.l., 59000 Lille (FR)
(72) Inventeur: BROUTIN, Pierre, 59000 Lille (FR)
(74) Mandataire: Argyma
(86) Numéro de dépôt international: PCT/FR2014/052650
(87) Numéro de publication internationale: WO 2015/055966

(56) Documents cités:
- HANSEN ET AL: "Screening of Dairy Cows for Ketosis by Use of Infrared Spectroscopy and Multivariate Calibration", JOURNAL OF DAIRY SCIENCE, AMERICAN DAIRY SCIENCE ASSOCIATION, US, vol. 82, no. 9, 1999, pages 2005-2010, XP026993664, ISSN: 0022-0302 [extrait le 1999-09-01]
- ENJALBERT F ET AL: "Ketone Bodies in Milk and Blood of Dairy Cows: Relationship between Concentrations and Utilization for Detection of Subclinical Ketosis", JOURNAL OF DAIRY SCIENCE, AMERICAN DAIRY SCIENCE ASSOCIATION, US, vol. 84, no. 3, 2001, pages 583-589, XP026990200, ISSN: 0022-0302 [extrait le 2001-03-01]
- DE ROOS A P W ET AL: "Screening for Subclinical Ketosis in Dairy Cattle by Fourier Transform Infrared Spectrometry", JOURNAL OF DAIRY SCIENCE, AMERICAN DAIRY SCIENCE ASSOCIATION, US, vol. 90, no. 4, 2007, pages 1761-1766, XP026955831, ISSN: 0022-0302 [extrait le 2007-04-01]
- OTO HANUS ET AL: "Milk acetone determination by the photometrical method after microdiffusion and via FT infra-red spectroscopy", JOURNAL OF AGROBIOLOGY, vol. 28, no. 1, 2011, XP055119188, ISSN: 1803-4403, DOI: 10.2478/v10146-011-0004-9
- ROBERT P ET AL: "MULTIVARIATE ANALYSIS APPLIED TO NEAR-INFRARED SPECTRA OF MILK", ANALYTICAL CHEMISTRY, AMERICAN CHEMICAL SOCIETY, US, vol. 59, no. 17, 1987, pages 2187-2191, XP000857119, ISSN: 0003-2700, DOI: 10.1021/AC00144A038
- Zhigang Zhang ET AL: "Detection of Subclinical Ketosis in Dairy Cows", Pakistan Veterinary Journal, mai 2012 (2012-05), pages 156-160, XP055176570, Extrait de l'Internet: URL:http://doaj.org/search?source=%7B%22qu ery%22%3A%7B%22bool%22%3A%7B%22must%22%3A% 5B%7B%22term%22%3A%7B%22id%22%3A%222a48d3a e1cc84a2d9265cd4cf8cff83b%22%7D%7D%5D%7D%7 D%7D [extrait le 2015-03-12]

## Description

La présente invention appartient au domaine des méthodes d'analyse des milieux aqueux complexes, et plus particulièrement à celui des analyses des fluides biologiques par les techniques spectrométriques.

Elle a pour objet un procédé de calibration d'un appareil de spectroscopie infrarouge pour déterminer la concentration d'un composé C1 dans un fluide F1 d'un animal, par exemple dans le sang, à partir des données spectroscopiques de composants se trouvant dans un autre fluide de l'animal (F2), par exemple dans son lait. L'invention a également pour objet une méthode de routine pour la détermination de la teneur d'un composé C1 dans le fluide F1 à partir d'un échantillon du fluide F2.

La méthode préconisée répond à une problématique de portée générale, en proposant de prédire les teneurs de différents composants d'un premier fluide à partir du dosage de composants dans un autre fluide. Ce dernier peut être plus facile d'accès que le premier fluide, facilitant ainsi la mise à disposition du praticien de données physiologiques complètes et fiables. C'est le cas par exemple des composants du sang, qui sont les indicateurs de nombreuses pathologies, et dont on souhaite connaître la concentration à partir du dosage de composants d'un autre fluide par exemple le lait ou l'urine. L'invention, dont la genèse était liée initialement à la recherche de solutions en réponse à la prévalence importante des cas de cétose chez la vache laitière, s'est rapidement avérée offrir une démarche d'intérêt plus général, applicable à des besoins variées.

L'acétonémie, aussi appelée cétose, est une maladie métabolique rencontrée surtout chez les vaches laitières fortes productrices en début de lactation. Dans l'une et l'autre de ses deux formes (clinique et subclinique), elle provoque des pertes économiques importantes en élevage laitier, en engendrant des chutes de production ainsi que des problèmes de fertilité et l'augmentation des maladies du péri-partum. Dans le cas de cétoses sub-cliniques en particulier, les vaches laitières perdent beaucoup de poids, mais il n'y a pas de chute de production comme dans le cas de cétoses cliniques. Cette acétonémie passe donc inaperçue. Un diagnostic précoce et fiable est donc important.

Pour compenser le déficit énergétique dû à la gestation puis à lactation, la vache mobilise ses graisses de réserve sous forme d'acides gras non estérifiés (AGNE). Ces acides gras sont captés par le foie pour y être stockés sous forme de triglycérides. Si le foie "normal" contient 1% de triglycérides, 15 jours avant vêlage, le niveau de triglycérides peut atteindre 5%. Durant le premier mois, en fonction du déficit énergétique et en l'absence de facteurs lipotrophes, cette infiltration graisseuse s'amplifie.

Or, les triglycérides ne sont pas solubles dans le sang. Ils ne peuvent sortir du foie et être transportés dans le sang que sous forme de lipoprotéines à très basse densité (ou VLDL pour Very Low Density lipoprotein). En principe ces lipoprotéines sont utilisées en grande partie pour la production laitière, mais en cas de cétose, et selon l'importance de la mobilisation (déficit énergétique) et l'insuffisance de facteurs lipotrophes permettant l'évacuation des triglycérides du foie, on assiste à une accumulation graisseuse dans le foie. Lorsque celle-ci est trop importante, le foie se débarrasse des acides gras non estérifiés en les oxydant et en produisant des corps cétoniques, à savoir de l'acétone, de l'acétoacétate, et du BHB (β-hydroxybutyrate), qui vont se retrouver dans le sang à des teneurs anormales, et également, bien que dans une moindre mesure, dans le lait. On considère qu'il y a acétonémie subclinique lorsque la teneur en BHB dans le sang est supérieure à 1,4 mmol/l.

Différentes méthodes ont été proposées dont certaines sont actuellement utilisées pour diagnostiquer les cétoses de la vache laitière ou détecter leur prévalence dans les troupeaux. Elles reposent sur la détermination de composés du sang, du lait ou parfois des urines de l'animal, qui subissent des variations lorsque la maladie est déclarée. Les composés recherchés comme révélateurs des cétoses sont les corps cétoniques, ou l'un d'eux plus particulièrement.

En effet, bien que la cétose engendre des modifications substantielles de la concentration des composants majeurs et mineurs du sang et du lait chez la vache laitière, et que les corps cétoniques soient des paramètres mineurs, faiblement concentrés, volatils et instables (notamment l'acétone et l'acétoacétate), le dosage de ces derniers est la voie principalement utilisée pour permettre de diagnostiquer une cétose. Le rapport matière grasse / taux protéique (MG/TP) est aussi couramment utilisé comme un indicateur d'acétonémie (MG/TP>1,5) mais cet indicateur reste peu fiable.

Plusieurs méthodes chimiques visent à déterminer la concentration en corps cétoniques dans le sang : chromatographie gazeuse, cinétique de réaction associée à une mesure d'absorbance, fluorimétrie (voir par exemple l'article "Detection of Subclinical Ketosis in Dairy Cows" de Zhigang Zhang et coll. dans Pakistan Veterinary Journal, mai 2012, pages 156-160). Ces analyses qui sont faites à partir d'échantillons sanguins nécessitent un prélèvement invasif et le recours à un laboratoire, avec des délais et des coûts élevés.

Une détermination quantitative des corps cétoniques à partir d'échantillons de lait est également possible. Les méthodes chimiques de laboratoire (test à la vanilline pour l'acétone, cinétique enzymatique de l'acétone et du BHB sur photomètre de la société SKALAR) sont décrites par Enjalbert et coll. dans J. Dairy Sci. 84:583-589. Elles ont également des limitations : bien qu'elles soient fiables même pour la détermination de composants mineurs du lait, elles sont assez onéreuses, lentes et difficiles à mettre en place en routine pour une analyse en masse. De plus, ces méthodes sont limitatives car elles ne permettent de doser qu'une molécule spécifique ou qu'un nombre de molécules limité, en l'occurrence les corps cétoniques, et ne prennent nullement en compte les variations de concentration des autres composants liés directement ou indirectement aux problèmes métaboliques recherchés. Il existe des méthodes qui peuvent être utilisées à la ferme pour tester le lait, le sang ou les urines, mais ce sont des méthodes semi-quantitatives (test coloré par bandelettes par exemple).

Les méthodes de spectroscopie IR, beaucoup plus rapides et moins onéreuses, se sont développées ces dernières années. Elles sont utilisées couramment pour déterminer la concentration de composés du lait, qu'il s'agisse de composés dits majeurs, tels que les matières grasses (MG), les matières protéiques (MP), le lactose ; ou de composés dits mineurs, par exemple la composition fine en acides gras, les fractions protéiques, l'urée, l'acide citrique mais aussi les corps cétoniques.

On peut citer par exemple la publication de Per Waaben Hansen "Screening of Dairy Cows for Ketosis by Uses of Infrared Spectroscopy and Multivariate Calibration" (J. Dairy Sci., 82:2005-2010), qui expose un procédé de détection des cétoses par dosage de l'acétone dans le lait. Des échantillons de lait sont utilisés pour réaliser une calibration fondée sur une corrélation entre, d'une part les valeurs de concentration de l'acétone du lait obtenues par une méthode de référence et d'autre part, les valeurs de concentration de l'acétone du lait obtenues par FT-IR. Le dosage de l'acétone dans le lait est fait en routine par référence à ces échantillons témoins. Dans J. Dairy Sci., 90:1761-1766, Roos et coll*.* adoptent une démarche similaire concernant le dosage du BHB du lait.

Cependant, l'emploi des techniques de spectroscopie IR pour le diagnostic à partir des prélèvements de lait de troubles métaboliques, et singulièrement en cas de cétoses, soulève un certain nombre de problèmes. En effet, la concentration des corps cétoniques dans le lait est généralement très faible, même en cas de pathologie. Selon différentes études, la limite tolérée de la concentration en BHB est de à 0,07 mmol/l (soit 7,3 mg/l) dans le lait, alors que la valeur maximale tolérée de BHB dans le sang est de 1,4 mmol/l (146 mg/l). Dans l'étude menée dans le cadre de la présente invention, la teneur en BHB dans le sang était en moyenne 12,5 fois plus élevée que celle du BHB dans le lait.

Pour obtenir une détection des très faibles concentrations en acétone et en acétoacétate dans les fluides par spectrométrie infrarouge, US 6385549 propose de réaliser une calibration d'un spectromètre FT-IR (spectromètre utilisant les techniques infrarouges à Transformée de Fourier) à partir d'un jeu d'échantillons parmi lesquels certains sont dépourvus ou quasiment dépourvus du composé pour lequel la calibration est réalisée. La concentration du composé recherché, à savoir l'acétone, est inférieure à la limite de détection théorique de l'appareil. Cette méthode suppose de disposer d'un grand nombre d'échantillons dont la composition en acétone et en acétoacétate est connue par des méthodes de références. La calibration est développée pour déterminer, dans le fluide analysé uniquement, la concentration du composé spécifique recherché. Difficulté supplémentaire, la grande volatilité de l'acétone et de l'acétoacétate nuit à la fiabilité du dosage, d'autant que ces analyses sont généralement réalisées à partir d'échantillons obtenus dans le cadre du contrôle laitier. Le délai entre le prélèvement et l'analyse peut être très variable (de 1 à 6 jours), de même que les conditions de stockage (allant communément de 4°C à 40°C), ce qui porte atteinte à l'intégrité des échantillons. Les résultats, et par conséquent le diagnostic, en sont significativement altérés.

Finalement, la méthode de référence ou méthode directe pour la détection de l'acétonémie chez les vaches reste jusqu'à présent la mesure du taux de BHB dans le sang (c'est le "gold standard"). Les dosimètres de laboratoire ou portables pour le dosage de la concentration du BHB sanguin fonctionnent sur le principe de la réaction d'oxydoréduction de Williamson, catalysée par le BHBDHase. Le courant généré à l'électrode est proportionnel à la quantité initiale de BHB. En cas d'acétonémie subclinique, le seuil utilisé est de 1.4 mmol/l (soit 145,74 mg/l), avec prélèvement sur un minimum de douze vaches. Cette méthode, la plus couramment utilisée, est invasive et nécessite l'immobilisation de l'animal. Elle demande en outre l'intervention d'une personne qualifiée tel qu'un vétérinaire. De plus, la variabilité journalière du BHB sanguin peut être très importante, ce qui implique que plusieurs mesures devraient être réalisées afin de connaître plus précisément l'évolution du BHB sanguin de chaque animal.

Les essais réalisés pour le dosage du BHB, non pas dans le sang mais dans le lait, ont montré que ce dosimètre présente une trop faible sensibilité du fait que les concentrations du BHB du lait sont souvent basses. La corrélation entre les concentrations de BHB sanguin et de BHB du lait est faible, surtout dans les plus hautes concentrations qui sont le marqueur de la maladie. Cette corrélation est aussi fortement affectée par les délais entre les deux mesures (sang et lait) réalisées dans le cadre du contrôle laitier, ainsi que par le mode de conservation. Aucune méthode satisfaisante n'est actuellement disponible.

Il existe donc un besoin d'offrir aux éleveurs une méthode systématique, non invasive, simple et rapide, permettant de diagnostiquer avec un degré de fiabilité élevé, les cétoses et les déficits énergétiques chez les animaux, en particulier chez les femelles allaitantes et notamment dans la période de lactation de 3 à 7 semaines.

La présente invention vise à répondre à ce besoin en proposant une méthode dans laquelle le diagnostic de l'acétonémie se fait à partir de mesures quantitatives réalisées sur le lait de l'animal. Pour ce faire, il a été imaginé de déterminer le taux de BHB (ou des corps cétoniques) du sang, à l'aide d'un spectre infrarouge réalisé sur un échantillon de lait. La méthode proposée consiste à rechercher l'existence de corrélations entre i) les caractéristiques de différentes zones spectrales du spectre IR obtenu pour le lait et ii) les variations de concentration d'un composé recherché dans le sang, et ceci sans connaître la composition des échantillons de lait. Ces corrélations sont identifiées par analyse multivariée (PLS, PCR, ANN, MLR...) et sont intégrées dans le modèle de prédiction. Ainsi, tous les composés du lait qui sont directement ou indirectement corrélés à la variation métabolique recherchée, sont pris en compte. Leur lien direct ou indirect avec le métabolisme concerné n'est pas nécessairement connu, le spectre IR fournissant une empreinte de l'état biologique et physiologique de l'animal lors du prélèvement de lait.

Il est apparu de manière inattendue qu'une prédiction rapide et fiable du taux de BHB dans le sang était possible à partir d'un spectre infrarouge réalisé sur un échantillon de lait, avec une précision suffisante pour la détection potentielle et systématique des cétoses ou des déficits énergétiques, y compris en routine dans le cadre du contrôle laitier, et avec une haute cadence analytique (jusqu'à 600 analyses/heure). Ce résultat a pu être obtenu grâce à un procédé de calibration particulier du spectromètre et au développement d'un modèle de calibration, selon l'invention. La méthode préconisée s'est révélée avoir une portée générale car elle permet de prédire les teneurs de différents composants d'un fluide corporel, à partir du dosage de composants dans un autre fluide, par exemple le lait ou l'urine, facilitant ainsi la mise à disposition du praticien de données physiologiques complètes et fiables. On peut désormais surveiller l'état et l'évolution d'un troupeau ou de bêtes individuelles, mieux fonder un diagnostic en cas de maladie ou de désordre métabolique et mettre en place des mesures préventives plutôt que curatives.

Un but de l'invention est ainsi de fournir une méthode par laquelle on détermine, à partir d'un échantillon d'un fluide d'un animal et de son spectre, le taux d'un composant (qui peut être un composé ou un groupe de composés) présent dans un autre fluide et qui est un indice d'un état normal ou problématique affectant l'animal. Plus particulièrement, un but de l'invention est d'utiliser les échantillons de lait collectés dans le cadre du contrôle laitier traditionnel pour déterminer le taux de BHB dans le sang d'un animal, sans avoir à effectuer de prélèvement sanguin. Il est souligné que les termes "échantillon" et "fluide" désignent des catégories différentes, pour bien distinguer ce qui relève de la nature des fluides et ce qui relève d'un prélèvement particulier issu d'un fluide donné.

La présente invention a ainsi pour objet un procédé de calibration d'un appareil de spectroscopie infrarouge pour réaliser des spectres en vue de déterminer la concentration d'un composant C1 dans un fluide F1 d'un animal selon la revendication 1. Les techniques basées sur la spectroscopie infrarouge dans le domaine du moyen infrarouge (fréquences de 1000 cm⁻¹ à 4000 cm⁻¹) sont déjà utilisées dans l'industrie agro-alimentaire et notamment dans l'industrie laitière pour déterminer la concentration des constituants majeurs présents dans les produits biologiques tels que le lait, qui sont par exemple les matières grasses, les protéines, le lactose, mais aussi celles des composants mineurs au nombre desquels l'urée, les acides organiques, les acides gras libres, ....

L'absorbance totale d'un échantillon est la somme des absorbances de ses composants individuels dans chacune de leurs plages d'absorption respectives. Toutefois, si on prend le lait comme exemple particulier, du fait de ses nombreux composants, les différents pics du spectre se recouvrent. La quantité de lumière absorbée dans une plage donnée n'est pas spécifique d'un seul composant, ce qui rend difficile, voire impossible, une interprétation directe de la réponse du spectromètre pour chaque composant et par suite empêche le calcul précis de la concentration d'un composant donné à partir de l'absorbance de l'échantillon.

Pour surmonter ce problème, on a généralement recours à une calibration faisant appel à un modèle mathématique permettant de séparer la contribution des différents composants à l'absorption à une fréquence donnée et de déterminer leur poids respectif dans le résultat de la mesure. Pour établir le modèle de calibration, on analyse des échantillons tests représentatifs du milieu, d'une part par une méthode d'analyse chimique classique, et d'autre part à l'aide d'un système spectrométrique, puis on définit les paramètres d'une relation mathématique entre les deux séries de résultats. Le modèle de calibration une fois établi, on l'applique au résultat des mesures spectrométriques pour déterminer la concentration d'un composant donné dans un échantillon, en partant de la quantité de radiations absorbées à des fréquences caractéristiques.

Ces méthodes sont connues en elles-mêmes et ne seront pas décrites plus précisément ici. L'homme du métier est en mesure de réaliser de tels modèles par les moyens connus de calibration multivariée à sa disposition. Les outils mathématiques applicables sont par exemple : régression de type multiple linéaire ou non linéaire (MLR ou MnLR), moindres carrés partiels (PLS), à composantes principales (PCR) ou à réseau de neurones artificiels (ANN), dont les abréviations en anglais figurent entre parenthèses. On dispose d'ors et déjà de tels modèles pour de nombreux composés. Le principe est que les spectres obtenus pour le fluide étudié, et la concentration d'un composant de ce même fluide, sont utilisés pour établir le modèle mathématique prédictif. Une fois établi, celui-ci permet de connaître la concentration d'un composant dans un échantillon quelconque dudit fluide, à partir du seul spectre infrarouge de cet échantillon.

De manière originale, selon l'invention, ce sont les spectres obtenus pour un fluide autre que celui contenant le composant recherché qui servent de base au calcul du modèle prédictif : c'est à partir des spectres d'absorption des seuls échantillons de fluide F2 qu'on définit un modèle prédictif de la concentration du composant C1 dans le fluide F1. Ainsi, à l'étape e), on se fonde sur au moins une des plages spectrales corrélées, pour calculer un modèle mathématique prédictif de la concentration du composant C1 dans le fluide F1.

On peut donc à l'étape e), sélectionner sur le spectre une ou plusieurs, et de préférence au moins trois plages spectrales corrélées, et utiliser lesdites plages spectrales des spectres d'absorption des échantillons de fluide F2 pour calculer ledit modèle mathématique prédictif de la concentration du composant C1 dans le fluide F1. Les plages corrélées que l'on sélectionne correspondent de préférence aux bandes spectrales de plus forte corrélation.

Les échantillons des fluides F1 et F2 proviennent d'animaux formant un lot représentatif, c'est-à-dire des animaux qui constituent un ensemble reflétant la diversité du troupeau dans son milieu (diversité génétique, d'alimentation, nombre de jours de lactation, saisons, climats...). Les critères pour établir un ensemble statistiquement représentatif sont connus de l'homme du métier. On ajoute que cet ensemble devra aussi être représentatif au regard de l'état métabolique motivant les mesures (par exemple, lors de la recherche d'un composant sanguin marqueur de troubles de la lactation, on sélectionnera un lot d'animaux comprenant des vaches allaitantes). Le mode et la fréquence de prélèvement doit aussi être en adéquation avec le problème à diagnostiquer. Cet aspect est apparu important pour obtenir des modèles prédictifs fiables. Dans le cadre de la présente invention, il a pu être mis en évidence qu'il existe des variations significatives du taux de BHB sanguin entre le matin et le soir, ou en fonction de l'alimentation de l'animal. Ceci est possible notamment grâce à la simplicité du prélèvement des échantillons qui peut se faire à l'occasion du contrôle laitier, et aussi bien le matin que le soir. La méthode utilisée a pu mettre en évidence et prendre en compte ces variations pour obtenir une calibration robuste et fiable, à partir des échantillons prélevés et d'échantillons composites (mélange de la traite du soir et du matin qui est l'échantillon normalement utilisé dans le cadre du contrôle laitier).

Le fluide F2 doit contenir des quantités décelables d'au moins un composant C2 qui a une relation directe ou indirecte avec une voie métabolique du composant C1. Un composant C2 peut être un produit ou un coproduit direct ou indirect du métabolisme du composant C1, c'est-à-dire un produit ou coproduit d'une réaction ou d'une chaîne de réactions où le composant C1 est impliqué. Dans certains cas, le composant C2 peut être identique au composant C1. En général, le composant C1 est en lien avec un état métabolique particulier éventuellement pathologique à diagnostiquer, dont il peut être la cause, ou au contraire une conséquence. Ce problème peut être aussi bien d'origine endogène (génétique, maladie, vache en état de gestation, déficit énergétique, nombre de lactations...), qu'exogène (alimentation, saison, luminosité...).

Les composants C2 peuvent être connus comme étant des produits dérivés de la voie métabolique du composant C1 et pouvant se trouver *in fine* dans le fluide F2, à des quantités décelables par spectroscopie infrarouge. Ils peuvent aussi ne pas avoir été identifiés comme tels. Le fait qu'une corrélation entre des composants C2 d'un fluide F2 et la concentration d'un composant C1 d'un fluide F1 soit trouvée à l'issue du procédé de calibration met en évidence l'existence d'un effet physiologique, même si on n'en connaît pas toujours ni le mécanisme. Au-delà, le procédé inventif peut conduire à découvrir l'existence d'un lien physiologique jusque là ignoré entre composés.

C'est un des avantages de la méthode selon l'invention qu'il ne soit pas nécessaire de connaître préalablement la nature du ou des composants C2 pour établir le modèle prédictif. Il n'est pas obligatoire non plus de les identifier durant la mise en oeuvre du procédé selon l'invention, bien que, comme on le verra plus loin, c'est possible et même particulièrement intéressant pour accroître la valeur prédictive du modèle et également pour découvrir de nouvelles relations physiologiques.

Le composant C1 est choisi parmi ceux qui sont en relation avec un état physiologique particulier de l'animal, qu'il soit normal ou pathologique. Sa présence en quantités plus ou moins élevées dans le fluide F1 est un indice de l'état de l'animal et son dosage est un moyen de surveillance des animaux et/ou de diagnostic d'une carence, d'un trouble pathologique, etc. La méthode de référence utilisée pour son dosage à l'étape c), est une méthode communément employée et généralement reconnue par les spécialistes comme étant la plus précise et fiable. Elle peut faire l'objet d'une norme.

On a vu qu'à l'étape d), on identifie sur les spectres d'absorption des échantillons de fluide F2 obtenus en c), les plages spectrales qui sont corrélées avec la concentration du composant C1 dans les échantillons de fluide F1. Les plages spectrales corrélées peuvent être identifiées en calculant le coefficient de corrélation à chaque longueur d'onde du spectre entre la valeur d'absorbance et la valeur de référence du composant C1. Selon une caractéristique préférée de l'invention, elles sont identifiées à l'aide d'algorithmes de régression multivariée, dans l'intervalle de fréquences allant de 650 cm⁻¹ à 4000 cm⁻¹. De préférence, lesdites plages spectrales corrélées sont choisies dans les intervalles de fréquences allant de 950 cm⁻¹ à 1620 cm⁻¹ et de 1680 cm⁻¹ à 3100 cm⁻¹, car on sait que la zone comprise entre 1620 cm⁻¹ et 1680 cm⁻¹ correspond à l'une des bandes d'absorption de l'eau qui est difficilement exploitable.

Selon une caractéristique préférée du procédé objet de l'invention, à l'étape a), les échantillons des fluides F1 et F2 dont on se munit à l'étape a) proviennent de prélèvements effectués simultanément sur chaque animal d'un lot d'animaux représentatifs, c'est-à-dire qu'ils sont prélevés sur chaque animal du lot dans un laps de temps réduit (par exemple dans la même heure) et en tous cas dans la même période du cycle circadien. Il est recommandé de conserver les échantillons en chambre froide et d'y ajouter un conservateur, afin que le fluide ne soit pas dénaturé avant de réaliser les analyses. De la sorte, le lien susceptible d'exister entre la composition des deux fluides à un instant t peut être mis en évidence en comparant les deux échantillons, et ce lien est préservé même si les analyses ne sont pas immédiates.

Selon une autre caractéristique préférée de l'invention, les étapes b) à e) sont réalisées avec les échantillons non dilués des fluides F1 et F2 prélevés à l'étape a). Il est en effet recommandé d'utiliser des échantillons naturels du fluide F2 afin de conserver la relation quantitative entre les composants de chacun des fluides F2 et F1. Par exemple, on utilisera des échantillons de lait naturel afin de maintenir le lien entre composition sanguine et composition du lait. L'adultération du lait avec du BHB ou d'autres produits ou coproduits directs ou indirects du métabolisme du composant C1 pour créer une variation artificielle, n'est pas recommandée car elle amoindrirait le lien de causalité.

Comme déjà mentionné, la méthode ci-dessus décrite peut être mise en oeuvre sans connaître la nature exacte des composants de F2 pour lesquels une corrélation est trouvée à l'étape d). Cependant, et c'est un avantage notable de la présente méthode, les spectres infrarouges obtenus à l'étape c) peuvent être utilisés de manière à identifier les composants C2 spécifiques du fluide F2 dont la concentration est en relation avec la concentration du composant C1 dans le fluide F1.

Il peut être commode pour identifier la nature du ou des composants C2, de mettre en relation le niveau de corrélation obtenu avec chaque fréquence spectrale. Lorsque les plages spectrales corrélées sont identifiées à l'étape d), on calcule le coefficient de corrélation (r) entre chaque point des spectres des échantillons du fluide F2 et les valeurs de référence de la concentration du composant C1 dosée dans le fluide F1. Lorsqu'on représente la variation des coefficients de corrélation en fonction de la fréquence, on obtient un graphe, appelé "spectre de corrélation", qui fait apparaître des maximums à certaines fréquences : ce sont les fréquences pour lesquelles la corrélation est la plus forte. Si on compare le spectre de corrélation avec les spectres infrarouges de différents composés répertoriés dans les bases de données, on voit apparaître pour certains des composés une superposition de pics (maximum d'absorbance) avec des plages de corrélation maximum : le composé en question absorbe fortement aux fréquences des plages fortement corrélées avec le composant C1. Ceci indique d'une part que ce composé particulier apporte une contribution importante au modèle de calibration, et d'autre part qu'il existe une relation entre le composant C1 et ce composé C2 particulier. C'est ainsi que nous avons pu mettre en évidence une corrélation significative entre le profil en acides gras du lait et certains acides gras avec le BHB du sang.

Certains composants du fluide F2 sont connus comme étant des produits obtenus lors des réactions métaboliques à partir du composant C1. Par exemple, on sait qu'en cas d'acétonémie, le taux de BHB dans le sang augmente, et qu'on retrouve du BHB dans le lait, mais aussi des acides gras et d'autres corps cétoniques. On va donc s'orienter vers ces composants connus comme étant des produits du métabolisme du BHB (composant C1) ou associés directement ou indirectement aux problèmes métaboliques qui sont à l'origine des variations de la concentration du BHB. On peut notamment établir le spectre de corrélation et le comparer avec les spectres infrarouges des différents acides gras, pour faire apparaître des correspondances de maximums.

On peut aussi rechercher un composant C2, dont on ne sait pas s'il est en relation métabolique avec le composant C1, mais pour lequel on constate que le spectre d'absorption présente une ou plusieurs bandes caractéristiques qui coïncident avec une ou plusieurs des bandes identifiées comme étant corrélées au composant C1. On va alors se fonder sur la morphologie de spectres répertoriés (dans des bases de données spectrales par exemple) pour sélectionner des composants C2 et déterminer une loi de corrélation entre leur concentration dans le fluide F2 et le composant C1 du fluide F1.

Ainsi, selon l'invention, le procédé de calibration comprend les étapes supplémentaires dans lesquelles :
f)- on recherche parmi les composants susceptibles d'être présents dans le fluide F2, au moins un composant C2 ayant au moins une fréquence d'absorption caractéristique qui coïncide avec une des bandes localisées sur le spectre pour lesquelles une corrélation existe,
g)- on détermine la concentration dudit au moins un composant C2 dans chacun des échantillons de fluide F2,
h)- on calcule la relation de corrélation R1 existant entre i) les concentrations dudit au moins un composant C2 dans le fluide F2 et ii) les concentrations du composant C1 dans le fluide F1.

On dispose ainsi d'un second modèle mathématique prédictif de la concentration du composant C1 dans le fluide F1, à partir des seuls spectres du fluide F2. Il va permettre de renforcer le pouvoir prédictif du procédé de calibration. Les composants susceptibles d'être présents dans le fluide F2 sont ceux que l'homme du métier connaît comme entrant dans la composition du fluide F2 des animaux sains ou malades. De manière particulièrement originale et fructueuse, l'étape f) va aussi permettre d'identifier la nature de composants C2 qui n'étaient pas jusqu'alors candidats à entrer dans la chaîne métabolique du composant C1.

Il est à noter que de manière alternative, on peut omettre l'étape f). Il est possible de déterminer la nature du ou des composants C2 corrélés à la teneur du composant C1 en calculant directement le coefficient de corrélation entre les valeurs de C2 dans le fluide F2 obtenues avec toutes les calibrations déjà à disposition et le composé C1. Cette étape permet de s'affranchir de l'étape f en recherchant et en identifiant, parmi les composants pour lesquels une calibration existe déjà (par exemple, profil complet en acides gras, acides organiques, fractions protéiques, glycoprotéines, glucides...), la nature du composé C2 pour lequel on a trouvé une corrélation.

Conformément à un mode de réalisation particulier du procédé selon l'invention, à l'étape g), la concentration dudit au moins un composant C2 dans les échantillons de fluide F2 peut être déterminée à partir des spectres infrarouges obtenus à l'étape c), à l'aide d'un modèle mathématique prédéterminé s'appliquant spécifiquement audit composant C2. C'est une démarche connue de l'homme de l'art, qui sait réaliser une calibration multivariée à partir d'une série de spectres infrarouges.

A noter que pour être exploitable, la concentration du composant C2 dans les échantillons du fluide F2 devra être déterminée en régime de routine, en même temps que la concentration des autres composants. L'analyseur infrarouge devra donc être équipé d'une calibration propre au composant C2 qui permettra d'obtenir la valeur de la concentration de C2 par analyse du spectre infrarouge du fluide F2. Si la nature de C2 est connue, on pourra utiliser des modèles prédictifs préexistants. Si cette calibration n'est pas déjà disponible, on prendra la précaution de doser la teneur du composant C2 dans le fluide F2 par la méthode de référence pour ce composant, et de développer le modèle correspondant.

La démarche ci-dessus exposée peut s'appliquer à un composant tel qu'un acide gras particulier, ou à un groupe de composants, par exemple aux acides gras saturés ou insaturés pris ensemble. Elle peut également prendre en compte des contributions combinées de plusieurs composants au travers d'un indicateur complexe. Dans ce cadre, les étapes f), g) et h) du procédé selon l'invention peuvent être réalisées de la manière suivante :
f')- on recherche parmi les composants susceptibles d'être présents dans le fluide F2, n composants C2n ayant chacun au moins une fréquence d'absorption caractéristique qui coïncide avec au moins une des bandes localisées sur le spectre pour lesquelles une corrélation existe,
g')- on détermine la concentration desdits n composants C2n dans chacun des échantillons de fluide F2,
h')- on calcule la valeur d'un indicateur Ic proportionnel à la concentration d'un au moins desdits n composants C2n,
h")- on calcule la relation de corrélation R2 existant entre i) les valeurs de l'indicateur Ic dans le fluide F2 et ii) les concentrations du composant C1 dans le fluide F1.

La définition d'un tel indicateur permet de prendre en compte différents composés se retrouvant dans le fluide F2 produits par une voie métabolique, toujours complexe, un seul composé pouvant être soumis à des variations ou à des influences variées. C'est le cas notamment lorsqu'une pathologie se déclare. Les composés marqueurs de cette pathologie peuvent se trouver à des concentrations très différentes dans les fluides F1 et F2 des animaux sains et des animaux malades.

L'indicateur Ic est proportionnel à la concentration d'un au moins desdits n composants C2n. Il peut ainsi être directement ou inversement proportionnel à la teneur d'un composant C2 déterminé. Il peut être un rapport des teneurs de deux composants C2a et C2b pour lesquels une corrélation existe, ou bien l'indicateur Ic peut être basé sur un composant C2n combiné à un autre composant non corrélé avec le composant C1. Un tel indicateur peut être par exemple le rapport entre les matières grasses et le taux protéique (MG/TP), ou encore le rapport entre certains acides gras et le taux protéique, etc.

Selon une caractéristique préférée de l'invention, la concentration desdits composants C2n dans les échantillons de fluide F2 est déterminée à partir des spectres infrarouges obtenus à l'étape c), à l'aide d'un modèle mathématique prédéterminé s'appliquant auxdits composants C2n. Comme indiqué précédemment, l'analyseur infrarouge devra être équipé d'un modèle de prédiction propre à chacun des composants C2n impliqué dans l'indicateur, préexistant ou s'il n'est pas disponible, développé à cette occasion par une méthode de calibration multivariée. Si besoin, il faudra utiliser la méthode de référence pour doser chaque composant C2n et développer les modèles correspondants.

Selon une caractéristique intéressante du procédé objet de l'invention, le fluide F1 est le sang d'un animal sain ou malade, et le fluide F2 est un autre fluide dudit animal sain ou malade, choisi parmi le lait ou l'urine. Le lot des animaux choisis sera constitué dans les règles de l'art, de manière à disposer d'un échantillonnage représentatif, pour une calibration robuste. Par exemple, pour la détection des cétoses, on sélectionnera les vaches de plusieurs élevages de la région concernée, et on prélèvera des échantillons de lait matin et soir sur toutes les vaches entre 3 et 7 semaines de lactation, plusieurs fois par semaine durant un mois. L'échantillonnage devra également être représentatif des différentes races d'animaux et pourra aussi prendre en compte les systèmes d'alimentation qui seront testés ensuite par le modèle.

Dans le procédé selon l'invention, le composant C1 est de préférence une substance dont la concentration dans le fluide F1 est un indice (direct ou indirect) d'un trouble ou d'un état métabolique particulier. Notamment, le composant C1 peut être un corps cétonique, un acide gras, un glucide, une protéine, une glycoprotéine, ou une hormone. On sait que les corps cétoniques sont des marqueurs de l'acétonémie. La glycémie sanguine est également souvent recherchée, ainsi que le taux d'hormones, notamment la progestérone. Des glycoprotéines sanguines comme la lactoferrine ou les glycoprotéines associées à la gestation (PAG) sont également recherchées. Ces analyses sont actuellement conduites sur échantillons sanguins, mais pourront être simplifiées ou améliorées par dosage indirect dans le lait de composés dérivés, grâce au procédé de calibration selon l'invention.

De manière préférée, selon l'invention, le composant C1 est le BHB (β-hydroxybutyrate), présent dans le sang d'un animal sain, en état de déficit énergétique ou en état d'acétonémie. En effet, le BHB est beaucoup plus stable que les autres corps cétoniques et présente de ce fait un plus grand intérêt : il est devenu le principal marqueur des cétoses. Cependant, comme indiqué plus haut son dosage dans le lait est peu fiable, car son taux dans le lait est bien plus faible que dans le sang (proche ou sous la limite de détection par spectroscopie moyen infrarouge). En outre, les taux de BHB et d'acétone du lait chutent très rapidement en l'absence de réfrigération, condition qui ne peut pas être remplie aisément dans le cadre du contrôle laitier.

Selon un mode de réalisation intéressant de l'invention, ledit au moins un composant C2 peut être choisi parmi les composants connus comme ayant une relation directe ou indirecte avec une voie métabolique du composant C1. Ils peuvent par exemple être connus comme étant des produits (directs ou indirects) du métabolisme du composant C1, ou associés (directement ou indirectement) aux problèmes métaboliques, dont on souhaite déterminer la concentration dans le fluide F1. Certains composants du fluide F2 sont connus comme étant des produits obtenus lors des réactions métaboliques à partir du composant C1. Le métabolisme concerné peut être normal ou pathologique, auquel cas le composant C2 est un marqueur du problème métabolique recherché. C2 est donc dans ce cas un produit d'une réaction ou d'une chaîne de réactions dont le composant C1 (ou le problème métabolique) est à l'origine. Les études sur le métabolisme animal fournissent des indices quant aux relations physiologiques susceptibles de fournir de telles relations. Cependant, jusqu'à présent, ces connaissances n'ont pas été mises à profit pour le dosage indirect des composés du sang (ou d'un autre fluide). Le ou les composés C2 sélectionnés sont analysés à partir d'un même spectre, en utilisant au moins une bande d'absorption du composant C2 concerné présent à des quantités décelables dans le fluide F2. Il va de soi qu'une quantité décelable dans le fluide F2, peut ne pas l'être dans le fluide F1 (ou de manière insuffisamment fiable et précise).

De manière alternative ou complémentaire, selon l'invention, ledit au moins un composant C2 peut être un composant nouvellement identifié à l'issue de l'étape f) comme ayant une relation directe ou indirecte avec une voie métabolique du composant C1. C'est un avantage notable de la présente méthode que de permettre d'identifier des composants dont la relation avec un problème métabolique était jusqu'à lors ignorée, et d'en déterminer la nature exacte, grâce aux spectres infrarouges obtenus à l'étape c).

Selon un mode de réalisation particulier du procédé objet de l'invention, ledit un au moins composant C2 est un composant présent dans le lait d'un animal sain, en état de déficit énergétique ou d'acétonémie, choisi parmi les matières grasses, les protéines, les glycoprotéines les glucides. Le composant C2 peut être notamment une famille d'acides gras ou un acide gras spécifique ; les protéines, une famille de protéines ou une protéine spécifique ; les glycoprotéines, une famille de glycoprotéines ou une glycoprotéine spécifique ; les glucides, une famille de glucide ou un glucide spécifique. Les bandes caractéristiques sur les spectres infrarouges de ces composés ou groupes de composés sont répertoriées, de sorte qu'une personne du métier sait exploiter les données spectrales les concernant. Par exemple, dans le cas de la détermination du BHB sanguin, le composant C2 peut être un corps cétonique, un acide gras ou une famille d'acides gras, dans la mesure où ces composés ont une relation avec la voie métabolique du BHB.

De préférence, selon l'invention, ledit un au moins composant C2 est un acide gras insaturé non estérifié, de préférence l'acide oléique ou l'acide stéarique. En effet, dans le cas de la détermination du BHB sanguin à partir du spectre du lait, nous avons établi qu'il existe une corrélation importante entre le taux de BHB sanguin et le profil en acides gras du lait (acides gras saturés, acides gras insaturés, mono et poly insaturés...) et tout particulièrement avec les acides gras insaturés, les acides oléique et stéarique.

Dans un mode de réalisation particulièrement intéressant de l'invention, l'indicateur Ic est défini comme étant le rapport entre le taux d'un acide gras ou d'une classe d'acides gras, (par exemple les acides gras saturés ou insaturés) et le taux protéique.

Le procédé décrit ci-dessus est destiné à paramétrer un appareil de spectroscopie infrarouge, en vue de réaliser des analyses de routine dans les élevages, ou des analyses dans le cadre d'études scientifiques. C'est pourquoi un autre objet de la présente invention est un procédé de détermination de la concentration d'un composé C1 dans un fluide F1 d'un animal, mettant en oeuvre un appareil de spectroscopie infrarouge pour réaliser des spectres d'absorption, procédé qui comprend l'utilisation d'un procédé de calibration dudit appareil de spectroscopie tel que décrit ci-avant, pour enregistrer (toutes) les informations spectrales d'un échantillon d'un fluide F2 dudit animal, différent du fluide F1, ledit fluide F2 contenant au moins un composant C2 ayant une relation directe ou indirecte avec une voie métabolique du composant C1.

Comme expliqué précédemment, le composant C1 peut être à l'origine de la voie métabolique aboutissant à C2, ou il peut résulter d'un problème métabolique particulier qui est recherché. On détermine directement la concentration de C1 dans le fluide F1, à l'aide du spectre réalisé avec l'échantillon de fluide F2, sans avoir identifié la nature du composant C2. Plusieurs composants C2, produits directs ou indirects du métabolisme du composant C1, peuvent être présents dans le fluide F2, et servir chacun, séparément ou ensemble, à la prédiction de la teneur de C1 dans le fluide F1, puisque tous les composants présents dans le fluide F2 laissent leur empreinte sur le spectre infrarouge.

On peut également déterminer la concentration d'un composant C1 dans un fluide F1 d'un animal, à partir de composants C2 du fluide F2, dont la nature a été préalablement identifiée. Selon cette variante de l'invention, le procédé de détermination de la concentration d'un composant C1 dans un fluide F1 d'un animal, mettant en oeuvre un appareil de spectroscopie infrarouge pour réaliser des spectres d'absorption, comprend l'utilisation d'un procédé de calibration dudit appareil de spectroscopie tel que décrit plus haut, pour mesurer dans un échantillon d'un fluide F2 dudit animal, différent du fluide F1, des quantités décelables d'au moins un composant C2 déterminé qui est un produit direct ou indirect du métabolisme du composant C1. Le composant C2 est ici prédéterminé dans sa nature, c'est-à-dire qu'il est déjà connu de l'art antérieurement ou bien qui a été identifié préalablement à la mise en oeuvre du présent procédé de détermination. Cette connaissance de la nature du composant C2 va orienter le choix des plages spectrales pertinentes utilisées pour la calibration préalable aux déterminations de routine, et en renforcer le pouvoir prédictif.

Est plus particulièrement objet de la présente invention, un procédé de détermination de la concentration en BHB dans le sang d'un animal, mettant en oeuvre un appareil de spectroscopie infrarouge pour réaliser des spectres d'absorption, le procédé comprenant l'utilisation d'un procédé de calibration d'un appareil de spectroscopie infrarouge, pour mesurer dans un échantillon de lait dudit animal une concentration décelable d'au moins un composant, choisi parmi les acides gras insaturés, de préférence l'acide oléique ou l'acide stéarique, et le taux protéique. On détermine ainsi la concentration en BHB sanguin directement à partir du spectre du lait.

Grâce à la méthode ici décrite, il est désormais aisé de réaliser un suivi systématique de l'état de santé des animaux ou de leur état physiologique dans le cadre de la prévention des troubles tels que l'acétonémie des vaches allaitantes. La détermination indirecte du taux de BHB sanguin peut être utilisée pour suivre l'évolution du taux de BHB sanguin d'un animal au cours du temps (d'un contrôle à un autre) et également pour comparer le taux de BHB d'un animal par rapport au taux de BHB du troupeau. Un animal en état d'acétonémie est ainsi rapidement repéré.

Le procédé inventif a de nombreuses applications. Par exemple, il peut être appliqué à la détermination de la concentration en glycoprotéines (PAG) du sang à partir d'une analyse spectroscopique d'un échantillon de son lait afin de déterminer rapidement, précocement et précisément la concentration en PAG du sang et donc son état de gestation.

Grâce au procédé selon l'invention, quatre approches sont désormais possibles :
1- Détermination directe du composant C1 de F1 à partir du spectre de F2 (lait) sur la base d'une calibration multivariée.
2- Identification de composants C2 corrélés directement ou indirectement au composant C1 pour prédire C1 directement ou en relation avec 1) afin d'améliorer le diagnostic du problème métabolique.
3- Utilisation de ratio de composants C2 (indicateur Ic) corrélés directement ou indirectement au composant C1 pour prédire C1 directement ou en relation avec 1) afin d'améliorer le diagnostic du problème métabolique.
4- Détermination directe du composant C1 dans F1 en relation avec (2) ou (3)

De manière générale, le procédé objet de la présente invention est beaucoup plus rapide et moins onéreux que les méthodes précédemment employées. Il permet des analyses non invasives et qui peuvent être systématisées et répétées fréquemment. Il permet une analyse simultanée de l'ensemble des composants du milieu soumis à la spectroscopie, contrairement aux méthodes chimiques conventionnelles. Il ouvre la possibilité d'élaborer un diagnostic complet (matière grasse, protéines, lactose, profile en acides gras du lait...).

La présente invention sera mieux comprise, et des détails en relevant apparaîtront, grâce à la description qui va être faite d'une de ses variantes de réalisation, en relation avec les figures annexées, dans lesquelles :
La fig. 1 représente la relation entre les valeurs de la concentration en BHB dans le sang obtenues par mesure chimique directe (méthode de référence), et les valeurs du BHB sanguin obtenues par un modèle de prédiction établi à partir du spectre IR de 696 échantillons de lait, selon l'invention.
La fig. 2 représente le développement de l'indicateur Acide Oléique (AO)/Taux Protéique (TP) pour la prédiction des cétoses à partir du spectre IR de 696 échantillons de lait.
La fig. 3 représente le développement de l'indicateur Acide Stéarique (AS)/Taux Protéique (TP) pour la prédiction des cétoses à partir du spectre IR de 696 échantillons de lait.
La fig. 4 représente l'utilisation du rapport MG/TP pour la prédiction des cétoses à partir du spectre IR de 696 échantillons de lait, (non satisfaisant).
La fig. 5 représente les coefficients de régression B du modèle, en fonction de la fréquence d'absorption.
La fig. 6 représente le spectre de corrélation montrant la variation entre i) les coefficients de corrélation entre BHB Sang et le spectre infrarouge du lait.
La fig. 7 montre la superposition du même spectre de corrélation avec le spectre infrarouge d'un échantillon de crème.
La fig. 8 est un spectre d'absorption infrarouge du BHB dans de l'eau à la concentration de 0,71 mmol/l (73,9 mg/l) et un spectre de lait.
La fig. 9 représente une carte à deux dimensions des seuils de détection pour les deux variables AO et TP.
La fig. 10 est un graphe mettant en évidence l'amélioration de la prédiction du BHB sanguin à l'échelle du troupeau.

### EXEMPLE 1

La démarche ci-après présentée a été mise en oeuvre pour réaliser la calibration d'un spectromètre, en vue déterminer la teneur en BHB dans le sang de vaches allaitantes, à partir des spectres infrarouges d'échantillons de lait.
**1)** On collecte au moins 100 échantillons de lait, de préférence entre 100 à 300 échantillons, chacun correspondant à une vache individuelle. L'échantillonnage est fait en tenant compte des systèmes d'alimentation variés, des races variées et du problème métabolique à détecter. Par exemple on sélectionnant des vaches saines et en état d'acétonémie. Idéalement, on collecte entre 300 et 1000 échantillons représentatifs de la zone de collecte.
**2)** Les échantillons de lait sont réfrigérés à 4°C immédiatement après le prélèvement afin de maintenir leur intégrité et éviter l'évaporation de certains composants. En effet, la concentration des corps cétoniques dans le lait décroît très rapidement, en quelques heures, en l'absence de réfrigération. Un conservateur y est ajouté afin de maintenir leur intégrité jusqu'à leur analyse.
**3)** En même temps que le lait, on prélève sur chaque vache un échantillon de sang, afin de maintenir le lien temporel/causal/métabolique entre la composition du lait et du sang et d'obtenir une corrélation optimale. On le réfrigère immédiatement.
**4)** On effectue les analyses de la teneur en BHB dans les échantillons de sang par la méthode de référence, en l'occurrence avec le lecteur OPTIUM XCEED®. Les valeurs obtenues sont stockées dans une mémoire d'une unité centrale.
**5)** On réalise le spectre complet (650-4000 cm⁻¹) des échantillons de lait sur un analyseur moyen infrarouge à transformée de Fourier (IRTF). Les échantillons sont d'abord chauffés dans un bain-marie à une température comprise entre 38°C et 42°C, pendant 20 minutes au maximum, afin d'homogénéiser la matière grasse dans le flacon. L'analyseur infrarouge est équipé d'une pompe haute pression et d'un homogénéisateur afin casser les globules gras, pour que leur diamètre soit inférieur à la plus petite longueur d'onde utilisée dans le développement du modèle de calibration. Ces conditions expérimentales sont importantes car la zone spectrale utilisée (2800-3000 cm⁻¹) pour déterminer le profil en acide gras du lait est très sensible à la taille des globules gras. Les spectres infrarouges sont enregistrés automatiquement dans l'unité centrale.
**6)** Les valeurs BHB obtenues par la méthode de référence sont éditées dans le logiciel de chimiométrie et sont confrontées aux spectres pour développer le modèle de calibration : définir les zones spectrales et les B coefficients de régression du modèle permettant de prédire la concentration en BHB du sang de l'animal à partir de son échantillon de lait.

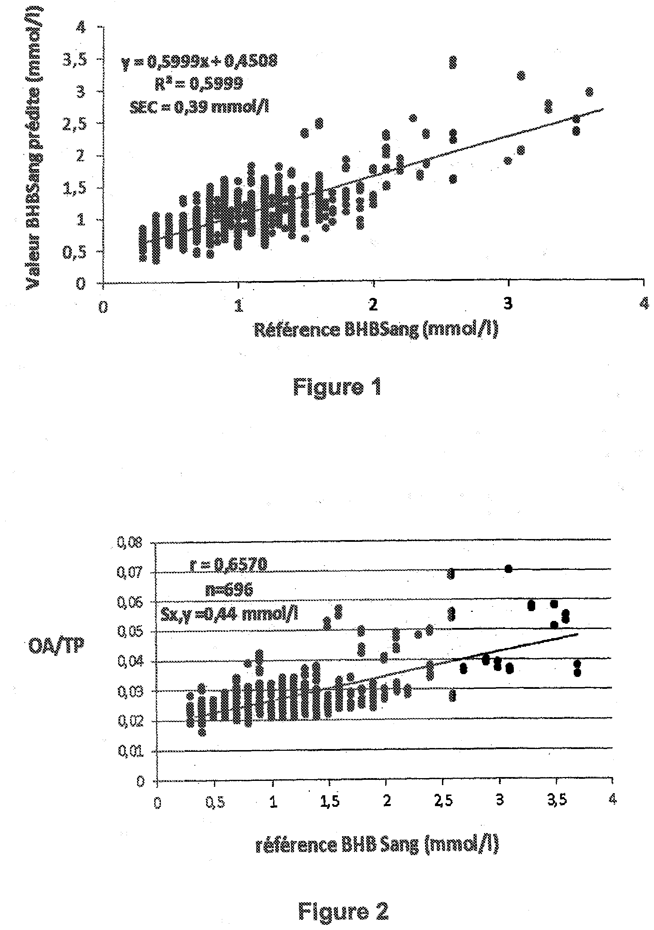

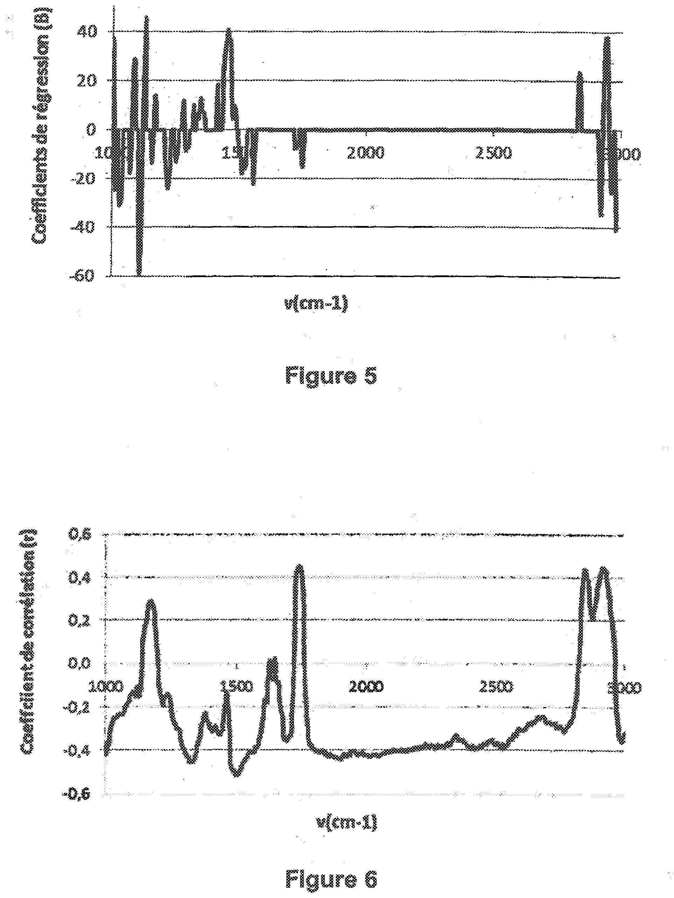

La fig. 5 représente les coefficients B du modèle. La fig. 1 illustre la valeur prédictive du modèle ainsi développé, en mettant en regard les valeurs des concentrations du BHB mesurées dans le sang avec celles obtenues à partir des spectres infrarouges du lait.

Il est à remarquer que le modèle de calibration a été développé sur la base d'échantillons de laits naturels, représentatifs des animaux atteints des désordres métaboliques qu'on cherchait à diagnostiquer. Une sélection préalable a été faite après étude approfondie de vaches représentatives dans plusieurs élevages de la région (< 100 jours de lactation). Le prélèvement des échantillons de lait dans s'est déroulé dans le cadre du contrôle laitier (matin et/ou soir) sur toutes les vaches sélectionnées, plusieurs fois par semaine au cours d'un mois (656 échantillons prélevés), avec remplissage complet des flacons d'analyse (pas de volume mort pour limiter l'évaporation). Les échantillons de sang ont été prélevés au niveau de la queue lors de chaque prélèvement de lait. On procède à la réfrigération immédiate des échantillons de lait à 4°C et on ajoute un conservateur tel que du bronopol (2-bromo-2-nitropropane-1,3-diol) afin de limiter l'évaporation des corps cétoniques.

### Résultats

Il est apparu qu'une prédiction rapide et fiable de la teneur en BHB du sang à partir d'un spectre infrarouge d'un échantillon de lait était possible. Le procédé ci-dessus a été mis en oeuvre sur 696 échantillons de lait de vache. Le modèle de calibration BHB du sang développé (voir fig.1) à partir des spectres infrarouges de lait et la concentration en BHB du sang donne un coefficient de corrélation (r) de 0,7370 et un écart type de prédiction de 0,39 mmol/l (40,6 mg/l). Cette précision est suffisante pour la détection potentielle et systématique des cétoses en routine dans le cadre du contrôle laitier à une cadence analytique maximale de 600 analyses/heure.

Nos travaux ont montré que la variation journalière (matin/soir) du taux de BHB sanguin pouvait être très importante. Le choix de l'échantillonnage a joué un rôle dans le succès du développement d'un modèle de calibration robuste qui intègre non seulement les variations de concentration des corps cétoniques mais aussi les variations de concentration des autres composants majeurs ou mineurs induites par le désordre métabolique ciblé, en particulier les variation de la composition en acides gras. Le spectre moyen infrarouge est une empreinte parfaite de la composition chimique et organique du lait et un miroir de l'état général de la vache. Il est donc très important de développer des modèles de calibration sur la base d'échantillons de laits naturels, représentatifs de vaches saines et de vaches atteintes de ces désordres métaboliques.

### EXEMPLE 2

Une identification des composants C2 du lait pour lesquels une corrélation existe avec la concentration en BHB sanguin a été effectuée. On a déterminé à partir des spectres des échantillons de lait, les zones spectrales qui sont corrélées avec la concentration en BHB du sang (et on les intégrées dans modèle de prédiction, comme expliqué à l'exemple 1).

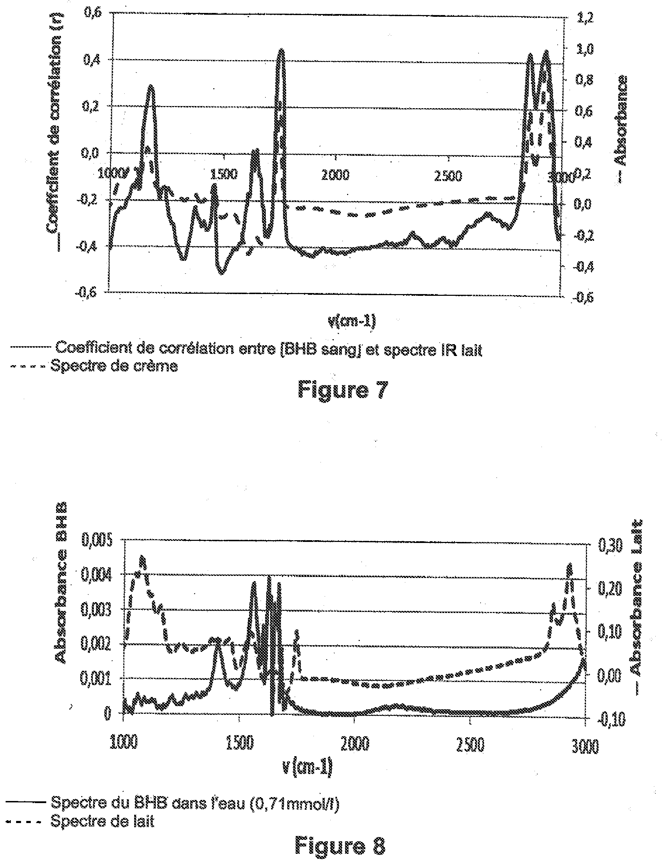

Pour déterminer la nature des composants C2 du lait (ou d'un indicateur Ic) corrélés à la teneur en BHB, la démarche adoptée a été la suivante :
- Calcul du coefficient de corrélation entre chaque point des spectres de lait et les valeurs de référence du BHB.
- Représentation graphique du "spectre de corrélation" (voir fig. 6) : il permet de visualiser quelles sont les zones spectrales corrélées au BHB du sang.
- Comparaison avec le spectre infrarouge de différents composants du lait. Le spectre d'un échantillon de crème a été mis en regard du spectre de corrélation (voir fig. 7).

Le "spectre de corrélation" obtenu met clairement en évidence les zones spectrales associées à la matière grasse et par conséquent au profil en acides gras du lait. Les bandes spectrales les mieux corrélées entre les spectres de lait et les valeurs de référence du BHB dans le sang correspondent aux principales bandes d'absorption des acides gras, à savoir 1170 cm⁻¹, 1380 cm⁻¹, 1450 cm⁻¹, 1750 cm⁻¹, 2850 cm⁻¹ et 2930 cm⁻¹. Le spectre de corrélation indique donc que le profil en acides gras du lait à un lien prépondérant (une variable explicative) direct ou indirect avec le taux de BHB sanguin. Ce résultat ne pouvait pas être obtenu directement à partir de la concentration en BHB dans le lait. En effet, comme on le voit sur la fig. 8, le spectre du BHB a deux bandes d'absorption charactéristiques à 1400 cm⁻¹ et 1560 cm⁻¹. Le spectre de corrélation entre les spectres de lait et le BHB Sang (fig. 8) ne met pas en évidence une correspondance particulière au niveau des pics charactéristiques du BHB. Le taux de BHB sanguin est beacoup plus corrélé à la composition en acides gras du lait qu'à la teneur du lait en BHB.

### EXEMPLE 3

Nous avons ensuite calculé les coefficients de corrélation des différents acides gras présents dans le lait et la concentration du BHB dans le sang. Cette dernière étape nous a permis de déterminer quels étaient les acides gras du lait les plus corrélés à la teneur en BHB du sang, et de les utiliser comme indicateurs pour affiner le diagnostic des cétoses ou d'un déficit énergétique.

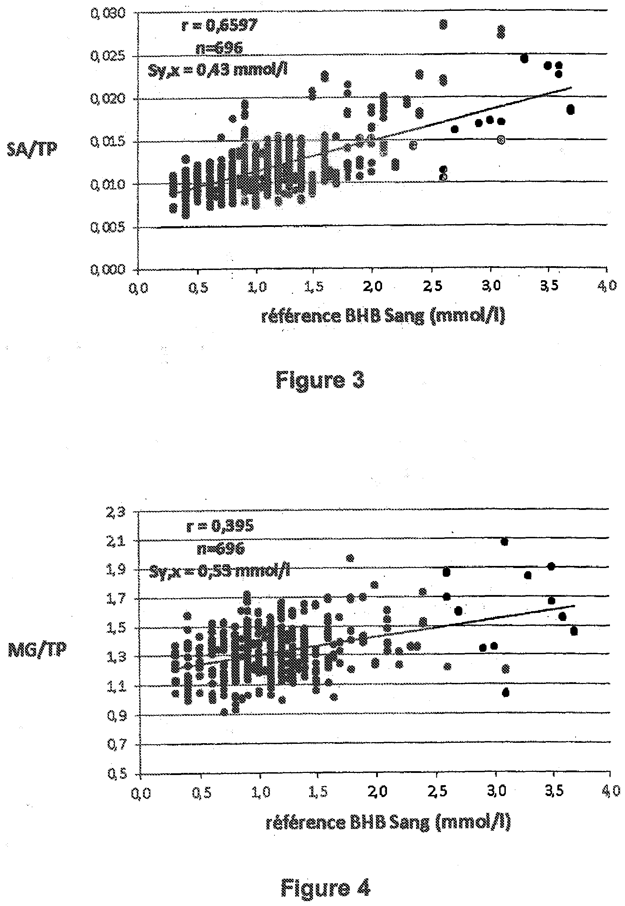

Les acides gras pour lesquels la corrélation était la plus forte sont l'acide oléique et l'acide stéarique. Si la présence d'acide oléique et sa corrélation à la teneur au BHB du sang était attendues pour des raisons physiologiques connues, l'intérêt d'autres acides gras, comme l'acide stéarique, a été mis en évidence par notre étude. Sur les 696 échantillons analysés, les coefficients de corrélation entre les taux d'acide oléique et acide stéarique avec le taux de BHB sanguin sont respectivement de 0,52 et 0,58. On voit ainsi que ces deux acides gras, et le profil en acide gras général du lait, ont un poids important dans le modèle de prédiction du BHB sanguin. La méthode selon l'invention a aussi permis de définir des indicateurs Ic plus performants. Elle a mis en évidence que les rapports AO/TP (taux d'acide oléique / taux protéique) et AS/TP (taux d'acide stéarique / taux protéique) étaient de meilleurs indicateurs de la cétose que le rapport MG/TP (matière grasse / taux protéique) utilisé actuellement. On précise qu'un rapport MG/TP > 1,5 est généralement indicateur d'une cétose. Sur les 696 échantillons analysés, nous avons obtenu une corrélation (r) entre AO/TP, AS/TP et les valeurs BHB du sang mesurées par le lecteur Optium Xceed® respectivement de 0,66 (fig.2) et 0,66 (fig.3). Par contre la corrélation enter MG/TP et les valeurs BHB du sang n'étaient que de 0,39 (fig.4). On constate donc une amélioration de 40,9% par rapport à l'indicateur MG/TP actuellement utilisé.

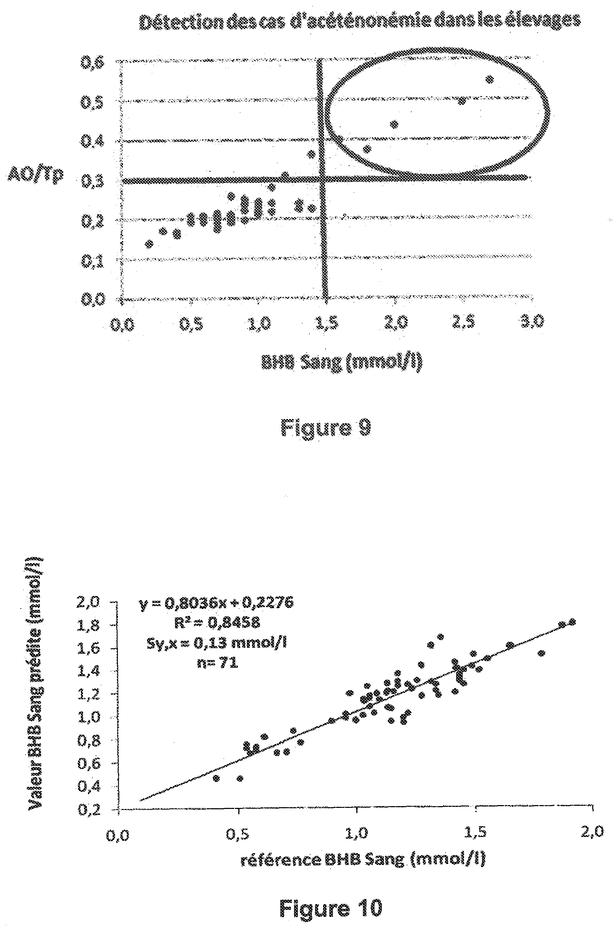

La détermination de certains composants C2 du lait corrélés directement ou indirectement au BHB (en l'occurrence de l'indicateur Ic = AO/TP) a permis d'affiner encore le diagnostic d'acétonémie. On peut en effet établir des seuils de détection pour les deux variables AO/TP et BHB, et mettre en forme une carte à deux dimensions comme montré à la figure 9. Les échantillons/animaux correspondant aux points du graphe entourés sont en état d'acétonémie. Les élevages disposent ainsi d'un outil de diagnostic de l'acétonémie.

### Conclusion

Les zones spectrales corrélées avec la concentration du composé recherché ont été directement identifiées par les algorithmes de régression multivariée (tels que PLS) avec le calcul des coefficients B de régression. Le calcul du spectre de corrélation peut être utilisé à la place des B coefficients, ou bien en complément, pour essayer de déterminer la nature du composant C2 corrélé au composant C1, en l'occurrence la nature de l'acide gras. Le spectre de corrélation a permis de mettre en évidence que c'est le profil en acide gras du lait qui était le plus corrélé au taux de BHB sang. La concentration des principaux acides gras du lait a ensuite été déterminée par des calibrations multivariées et on a déterminé celui/ceux qui étaient les plus corrélés au BHB du sang.

### EXEMPLE 4

On a vu que la détection des corps cétoniques dans le lait est une méthode peu fiable, dont l'objectif est uniquement de déterminer la concentration de ce composé dans le lait, et ne prend en compte que cette variable pour essayer d'expliquer le trouble métabolique. Au contraire, l'invention repose sur une approche qui prend en compte toutes les variables afin d'établir un lien direct entre la composition du lait et la concentration du BHB (composant C1) dans le sang. Le modèle prédictif intègre les traces de corps cétoniques présentes éventuellement dans le lait, mais surtout toutes les variables et données qui sont liées directement ou indirectement au BHB sanguin et au problème métabolique à diagnostiquer. De ce fait, la prédiction en est significativement améliorée.

Par exemple, l'amélioration de la prédiction du BHB sanguin à l'échelle du troupeau ou par groupe de vaches a été mise en évidence en calculant la moyenne de la référence et des valeurs prédites par troupeau. Les résultats sont reportés sur le graphe présenté à la figure 10.

Le niveau d'alerte pour le BHB sang est typiquement de 1,4 mmol/l. Les troupeaux ayant un problème d'acétonémie ou de déficit énergétique peuvent ainsi être facilement détectés grâce à notre méthode.

## Revendications

1. Procédé de calibration d'un appareil de spectroscopie infrarouge destiné à réaliser des spectres en vue de déterminer la concentration d'un composant C1 dans un fluide F1 d'un animal, ledit procédé étant **caractérisé en ce qu'**il comprend les étapes consistant à :
a)- se munir d'échantillons dudit fluide F1 et d'un fluide F2 différent du fluide F1, provenant de chaque animal appartenant à un lot d'animaux représentatifs, le fluide F2 contenant des quantités décelables d'au moins un composant C2 qui est un produit ou un coproduit direct ou indirect du métabolisme du composant C1,
b)- mesurer la concentration du composant C1 dans les échantillons de fluide F1 à l'aide d'une méthode de référence, et enregistrer les valeurs de référence obtenues dans une mémoire d'une unité centrale,
c)- réaliser le spectre d'absorption IR complet des échantillons de fluide F2 dans l'intervalle de fréquences allant de 650 cm⁻¹ à 4000 cm⁻¹, et enregistrer les spectres obtenus dans une mémoire de ladite unité centrale,
d)- Calculer le coefficient de corrélation à chaque longueur d'onde des spectres d'absorption obtenus en c) entre les valeurs d'absorbance des échantillons de fluide F2 et les valeurs de référence du composant C1 et identifier les plages spectrales pour lesquelles les valeurs d'absorbance sont corrélées avec les valeurs de référence de la concentration du composant C1 dans les échantillons de fluide F1, et
e)- calculer en se fondant sur au moins une desdites plages spectrales des spectres d'absorption des échantillons de fluide F2, un modèle mathématique prédictif de la concentration du composant C1 dans le fluide F1.

2. Procédé selon la revendication 1, **caractérisé en ce que** à l'étape d), lesdites plages spectrales corrélées sont identifiées à l'aide d'algorithmes de régression multivariée, dans l'intervalle de fréquences allant de 650 cm⁻¹ à 4000 cm⁻¹.

3. Procédé selon l'une des revendications précédentes, **caractérisé en ce que** les échantillons des fluides F1 et F2 dont on se munit à l'étape a) proviennent de prélèvements effectués simultanément sur chaque animal d'un lot d'animaux représentatifs.

4. Procédé selon l'une des revendications précédentes, **caractérisé en ce qu'**il comprend les étapes supplémentaires dans lesquelles :
f)- on recherche parmi les composants susceptibles d'être présents dans le fluide F2, au moins un composant C2 ayant au moins une fréquence d'absorption caractéristique qui coïncide avec une des bandes localisées sur le spectre pour lesquelles une corrélation existe,
g)- on détermine la concentration dudit au moins un composant C2 dans chacun des échantillons de fluide F2,
h)- on calcule la relation de corrélation R1 existant entre i) les concentrations dudit au moins un composant C2 dans le fluide F2 et ii) les concentrations du composant C1 dans le fluide F1.

5. Procédé selon l'une des revendications précédentes, **caractérisé en ce qu'**il comprend les étapes supplémentaires dans lesquelles :
f')- on recherche parmi les composants susceptibles d'être présents dans le fluide F2, n composants C2n ayant chacun au moins une fréquence d'absorption caractéristique qui coïncide avec au moins une des bandes localisées sur le spectre pour lesquelles une corrélation existe,
g')- on détermine la concentration desdits n composants C2n dans chacun des échantillons de fluide F2,
h')- on calcule la valeur d'un indicateur Ic proportionnel à la concentration d'un au moins desdits n composants C2n,
h")- on calcule la relation de corrélation R2 existant entre i) les valeurs de l'indicateur Ic dans le fluide F2 et ii) les concentrations du composant C1 dans le fluide F1.

6. Procédé selon l'une quelconque des revendications précédentes, **caractérisé en ce que** le fluide F1 est le sang d'un animal sain ou malade, et le fluide F2 est un autre fluide dudit animal sain ou malade, choisi parmi le lait, l'urine.

7. Procédé selon l'une des revendications précédentes, **caractérisé en ce que** le composant C1 est une substance dont la concentration dans le fluide F1 est un indice direct ou indirect d'un trouble métabolique ou d'un état métabolique particulier.

8. Procédé selon la revendication précédente, **caractérisé en ce que** le composant C1 est un corps cétonique, un acide gras, un glucide, une protéine, une glycoprotéine ou une hormone.

9. Procédé selon la revendication précédente, **caractérisé en ce que** le composant C1 est le BHB (β-hydroxybutyrate), présent dans le sang d'un animal sain, en état de déficit énergétique ou en état d'acétonémie.

10. Procédé selon une des revendications 4 à 9, **caractérisé en ce que** ledit au moins un composant C2 est un composant, connu ou nouvellement identifié à l'issue de l'étape f), comme ayant une relation directe ou indirecte avec une voie métabolique du composant C1.

11. Procédé selon l'une quelconque des revendications précédentes, **caractérisé en ce que** ledit un au moins composant C2 est un composant présent dans le lait d'un animal sain, en état de déficit énergétique ou d'acétonémie, choisi parmi les acides gras, les protéines, les glycoprotéines, les glucides.

12. Procédé selon la revendication précédente, **caractérisé en ce que** ledit un au moins composant C2 est un acide gras insaturé non estérifié, de préférence l'acide oléique ou l'acide stéarique.

13. Procédé selon la revendication 5, **caractérisé en ce que** l'indicateur Ic est défini comme étant le rapport entre le taux d'un acide gras ou d'une classe d'acides gras et le taux protéique.

14. Procédé de détermination de la concentration d'un composant C1 dans un fluide F1 d'un animal à l'aide d'un appareil de spectroscopie apte à réaliser des spectres d'absorption, **caractérisé en ce qu'**il comprend :
- d'utiliser un procédé de calibration dudit appareil de spectroscopie selon l'une des revendications 1 à 13, ledit modèle étant prédictif de ladite concentration C1 à partir d'un spectre d'un échantillon d'un fluide F2 dudit animal, différent du fluide F1, le fluide F2 contenant des quantités décelables d'au moins un composant C2 qui est un produit ou un coproduit direct ou indirect du métabolisme du composant C1,
- de réaliser un spectre d'un échantillon du fluide F2 dudit animal et enregistrer les informations spectrales dudit échantillon,
- d'appliquer le modèle mathématique aux informations spectrales de l'échantillon de fluide F2 pour déterminer la concentration du composant C1 dans le fluide F1.

15. Procédé selon la revendication précédente, dans lequel on détermine la concentration du BHB dans le sang d'un animal en appliquant ledit modèle mathématique aux informations spectrales obtenues à partir d'un échantillon de lait dudit animal.

## Patentansprüche

1. Verfahren zur Kalibrierung eines Infrarot-Spektroskopie-Geräts, das dazu bestimmt ist, Spektren aufzuzeichnen, um die Konzentration eines Bestandteils C1 in einer Flüssigkeit F1 zu bestimmen, welche von einem Tier stammt, wobei das Verfahren **dadurch gekennzeichnet ist, dass** es die folgenden Schritte umfasst:
a) - Bereitstellen von Proben der Flüssigkeit F1 sowie einer Flüssigkeit F2, die sich von der Flüssigkeit F1 unterscheidet, wobei diese von jeweils einem Tier stammt, das einer Gesamtheit repräsentativer Tiere angehört, wobei die Flüssigkeit F2 nachweisbare Mengen mindestens eines Bestandteils C2 enthält, bei welchem es sich um ein direktes oder indirektes Produkt oder Nebenprodukt der Verstoffwechslung des Bestandteils C1 handelt,
b) - Messen der Konzentration des Bestandteils C1 in den Proben der Flüssigkeit F1 mittels einer Referenzmethode, und Speichern der erhaltenen Referenzwerte in einem Speicher eines Zentralrechners,
c) - Aufzeichnen des vollständigen IR-Absorptionsspektrums der Proben der Flüssigkeit F2 im Frequenzintervall von 650 cm⁻¹ bis 4000 cm⁻¹, und Speichern der erhaltenen Spektren in einem Speicher des Zentralrechners,
d) - Berechnen des Koeffizienten, bei jeder der Wellenlängen der Absorptionsspektren, welche in c) erhalten wurden, der Korrelation zwischen den Absorptionswerten der Proben der Flüssigkeit F2 und den Referenzwerten des Bestandteils C1, und Identifizieren der spektralen Bereiche, in welchem die Absorptionswerte mit den Referenzwerten der Konzentration des Bestandteils C1 in den Proben der Flüssigkeit F1 korrelieren, und
e) - Berechnen, auf Grundlage mindestens eines dieser spektralen Bereiche der Absorptionsspektren der Proben der Flüssigkeit F2, eines mathematischen Modells zur Vorhersage der Konzentration des Bestandteils C1 in der Flüssigkeit F1.

2. Verfahren nach Anspruch 1, **dadurch gekennzeichnet, dass** im Schritt d) die korrelierenden spektralen Bereiche innerhalb des Frequenzintervalls von 650 cm⁻¹ bis 4000 cm⁻¹ mit Hilfe von Algorithmen der multivarianten Regression identifiziert werden.

3. Verfahren nach einem der vorhergehenden Ansprüche, **dadurch gekennzeichnet, dass** die Proben der Flüssigkeiten F1 und F2, die im Schritt a) bereitgestellt werden, aus Probennahmevorgängen stammen, die gleichzeitig bei jeweils einem Tier einer Gesamtheit repräsentativer Tiere durchgeführt wurden.

4. Verfahren nach einem der vorhergehenden Ansprüche, **dadurch gekennzeichnet, dass** es die folgenden zusätzlichen Schritte umfasst, welche darin bestehen:
f) - unter den Bestandteilen, die möglicherweise in der Flüssigkeit F2 vorliegen könnten, mindestens einen Bestandteil C2 ausfindig zu machen, welcher mindestens eine charakteristische Absorptionsfrequenz hat, die mit einer der Banden übereinstimmt, welche sich in dem Spektrum befinden und für welches eine Korrelation besteht,
g) - die Konzentration des mindestens einen Bestandteils C2 in jeder der Proben der Flüssigkeit F2 zu bestimmen,
h) die Korrelationsbeziehung R1 zu berechnen, wie sie zwischen i) den Konzentrationen des mindestens einen Bestandteils C2 in der Flüssigkeit F2 und ii) den Konzentrationen des Bestandteils C1 in der Flüssigkeit F1 besteht.

5. Verfahren nach einem der vorhergehenden Ansprüche, **dadurch gekennzeichnet, dass** es die folgenden zusätzlichen Schritte umfasst, welche darin bestehen:
f') - unter den Bestandteilen, die möglicherweise in der Flüssigkeit F2 vorliegen könnten, n Bestandteile C2n ausfindig zu machen, von denen jeder mindestens eine charakteristische Absorptionsfrequenz hat, die mit mindestens einer der Banden übereinstimmt, welche sich in dem Spektrum befinden und für welches eine Korrelation besteht,
g') - die Konzentration der n Bestandteile C2n in jeder der Proben der Flüssigkeit F2 zu bestimmen,
h') - den Wert einer Kennzahl Ic zu berechnen, die proportional zur Konzentration mindestens eines der n Bestandteile C2n ist,
h") - die Korrelationsbeziehung R2 zu berechnen, wie sie zwischen i) den Werten der Kennzahl Ic in der Flüssigkeit F2 und ii) den Konzentrationen des Bestandteils C1 in der Flüssigkeit F1 besteht.

6. Verfahren nach einem beliebigen der vorhergehenden Ansprüche, **dadurch gekennzeichnet, dass** es sich bei der Flüssigkeit F1 um das Blut eines gesunden oder kranken Tieres handelt und dass es sich bei der Flüssigkeit F2 um eine andere Flüssigkeit des gesunden oder kranken Tieres handelt, wobei diese aus der Milch und dem Urin ausgewählt ist.

7. Verfahren nach einem der vorhergehenden Ansprüche, **dadurch gekennzeichnet, dass** es sich bei dem Bestandteil C1 um eine Substanz handelt, deren Konzentration in der Flüssigkeit F1 auf direkte oder indirekte Weise eine Stoffwechselstörung oder einen bestimmten Stoffwechselzustand anzeigt.

8. Verfahren nach dem vorhergehenden Anspruch, **dadurch gekennzeichnet, dass** es sich bei dem Bestandteil C1 um einen Ketonkörper, eine Fettsäure, ein Kohlenhydrat, ein Protein, ein Glykoprotein oder ein Hormon handelt.

9. Verfahren nach dem vorhergehenden Anspruch, **dadurch gekennzeichnet, dass** es sich bei dem Bestandteil C1 um BHB (β-Hydroxybutyrat) handelt, das im Blut eines Tieres vorliegt, welches gesund ist, sich in einem Zustand des Energiemangels befindet oder sich in einem Zustand der Acetonämie befindet.

10. Verfahren nach einem der Ansprüche 4 bis 9, **dadurch gekennzeichnet, dass** es sich bei dem mindestens einen Bestandteil C2 um einen Bestandteil handelt, der bekanntermaßen, oder wie nach Abschluss des Schrittes f) neu erkannt wurde, in direkter oder indirekter Beziehung mit einem Stoffwechselweg des Bestandteils C1 steht.

11. Verfahren nach einem beliebigen der vorhergehenden Ansprüche, **dadurch gekennzeichnet, dass** es sich bei dem mindestens einen Bestandteil C2 um einen Bestandteil handelt, der in der Milch eines Tieres vorliegt, welches gesund ist, sich in einem Zustand des Energiemangels befindet oder sich einem Zustand der Acetonämie befindet, wobei er aus den Fettsäuren, den Proteinen, den Glykoproteinen, den Kohlenhydraten ausgewählt ist.

12. Verfahren nach dem vorhergehenden Anspruch, **dadurch gekennzeichnet, dass** es sich bei dem mindestens einen Bestandteil C2 um eine unveresterte ungesättigte Fettsäure handelt, vorzugsweise um Ölsäure oder Stearinsäure.

13. Verfahren nach Anspruch 5, **dadurch gekennzeichnet, dass** die Kennzahl Ic als Verhältnis zwischen dem Gehalt an einer Fettsäure oder an einer Kategorie von Fettsäuren und dem Gehalt an Proteinen definiert ist.

14. Verfahren zur Bestimmung der Konzentration eines Bestandteils C1 in einer Flüssigkeit F1 eines Tieres mit Hilfe eines Spektroskopie-Geräts, das dazu befähigt ist, Absorptionsspektren aufzuzeichnen, **dadurch gekennzeichnet, dass** es Folgendes umfasst:
- Anwenden eines Verfahrens zum Kalibrieren des Spektroskopie-Geräts nach einem der Ansprüche 1 bis 13, wobei das Modell die Konzentration C1 ausgehend von einem Spektrum einer Probe einer Flüssigkeit F2 des Tieres vorhersagt, wobei diese sich von der Flüssigkeit F1 unterscheidet, wobei die Flüssigkeit F2 nachweisbare Mengen mindestens eines Bestandteils C2 enthält, bei welchem es sich um ein direktes oder indirektes Produkt oder Nebenprodukt der Verstoffwechslung des Bestandteils C1 handelt,
- Aufzeichnen eines Spektrums einer Probe der Flüssigkeit F2 des Tieres und Speichern der spektralen Information der Probe,
- Anwenden des mathematischen Modells auf die spektrale Information der Probe der Flüssigkeit F2, um die Konzentration des Bestandteils C1 in der Flüssigkeit F1 zu bestimmen.

15. Verfahren nach dem vorhergehenden Anspruch, wobei die Konzentration an BHB im Blut eines Tieres bestimmt wird, indem das mathematische Modell auf die spektrale Information angewendet wird, welche ausgehend von einer Milchprobe des Tieres erhalten wurde.

## Claims

1. Method for calibrating an infrared spectroscopy apparatus in order to produce spectra with a view to determining the concentration of a component C1 in a fluid F1 of an animal, said method being **characterized in that** it comprises the following steps:
a) obtaining samples of said fluid F1 and of a fluid F2 other than the fluid F1 from each animal belonging to a group of representative animals, the fluid F2 containing detectable amounts of at least one component C2 which is a product or a direct or indirect coproduct of the metabolism of the component C1,
b) measuring the concentration of the component C1 in the samples of fluid F1 by means of a reference method, and recording the obtained reference values in a memory of a central unit,
c) producing the complete IR absorption spectrum of the sample of fluid F2 in the frequency range from 650 cm⁻¹ to 4000 cm⁻¹, and recording the obtained spectra values in a memory of a central unit,
d) calculating the correlation coefficient at each wavelength of the absorption spectra obtained in c), between the absorption values of the samples of fluid F2 and the reference values of the component C1 and identifying the spectral ranges for which the absorption values are correlated with the reference values of the concentration of the component C1 in the samples of fluid F1, and
e) calculating, on the basis of at least one of said spectral ranges of the absorption spectra of the samples of fluid F2, a predictive mathematical model of the concentration of the component C1 in the fluid F1.

2. Method according to claim 1, **characterized in that** at step d), said correlated spectral ranges are identified by means of multivariate regression algorithms, in the interval of frequencies ranging from 650 cm⁻¹ to 4000 cm⁻¹.

3. Method according to one of the preceding claims, **characterized in that** the samples of the fluids F1 and F2 obtained in step a) come from samples taken simultaneously from each animal in a group of representative animals.

4. Method according to one of the preceding claims, **characterized in that** it comprises the following additional steps:
f)- searching, among the components likely to be present in the fluid F2, for at least one component C2 having at least one characteristic absorption frequency that coincides with one of the bands located on the spectrum for which a correlation exists,
g)- determining the concentration of said at least one component C2 in each of the samples of fluid F2,
h)- calculating the correlation relation R1 existing between i) the concentrations of said at least one component C2 in the fluid F2 and ii) the concentrations of the component C1 in the fluid F1.

5. Method according to one of the preceding claims, **characterized in that** it comprises the following additional steps:
f')- seeking among the components likely to be present in the fluid F2, n components C2n, each having at least one characteristic absorption frequency that coincides with at least one of the bands located in the spectrum for which a correlation exists,
g')- determining the concentration of said n components C2n in each of the samples of the fluid F2,
h')- calculating the value of an indicator Ic proportional to the concentration of at least one of said n components C2n,
h")- calculating the correlation relation R2 existing between i) the values of the indicator Ic in the fluid F2 and ii) the concentrations of the component C1 in the fluid F1.

6. Method according to any of the preceding claims, **characterized in that** the fluid F1 is the blood of a healthy or diseased animal, and the fluid F2 is another fluid of said healthy or diseased animal, either milk or urine.

7. Method according to any of the preceding claims, **characterized in that** the component C1 is a substance whose concentration in the fluid F1 is a direct or indirect indication of a disorder or particular metabolic state.

8. Method according to the preceding claim, **characterized in that** the component C1 is a ketone body, a fatty acid, a carbohydrate, a protein, a glycoprotein or a hormone.

9. Method according to the preceding claim, **characterized in that** the component C1 is BHB (β-hydroxybutyrate), present in the blood of a healthy animal, in a state of energy deficit or in a state of acetonemia.

10. Method according to one of claims 4 to 9, **characterized in that** said at least one component C2 is chosen from components known or newly identified on completion of step f), as having a direct or indirect relation with a metabolic pathway of the component C1.

11. Method according to any of the preceding claims, **characterized in that** said at least one component C2 is a component present in the milk of a healthy animal, in a state of energy deficit or acetonemia, chosen from fatty acids, proteins, glycoproteins and carbohydrates.

12. Method according to the preceding claim, **characterized in that** said at least one component C2 is an unsaturated non-esterified fatty acid, preferably oleic acid or stearic acid.

13. Method according to claim 5, **characterized in that** the indicator Ic is defined as being the ratio between the level content of a fatty acid or a class of fatty acids and the protein content.

14. Method for determining the concentration of a component C1 in a fluid F1 of an animal, using a spectroscopy apparatus to produce absorption spectra, **characterized in that** it comprises:
- using a method of calibrating said spectroscopy apparatus according to one of claims 1 to 13, said model being predictive of said concentration C1 from a spectrum of a sample of a fluid F2 of said animal, other than the fluid F1, the fluid F2 containing detectable amounts of at least one component C2 which is a product or a direct or indirect coproduct of the metabolism of the component C1,
- producing a spectrum of a sample of fluid F2 of said animal and recording the spectral information of said sample,
- applying the mathematical model to the spectral information of the sample of fluid F2 to determine the concentration of the component C1 in the fluid F1.

15. Method according to the preceding claim, wherein the concentration of BHB in the blood of an animal, is determining by applying said mathematical model to the spectral information obtained from a sample of milk of said animal.
